# DEMANDE DE BREVET EUROPEEN

(11) **EP 1 671 677 A1**
(43) Date de publication de la demande: **21.06.2006**
(21) Numéro de dépôt: 05292562.5
(22) Date de dépôt: 02.12.2005
(51) Int. Cl.: A61Q 17/04

(54) **Composition solaire comprenant au moins des pigments mineraux d'oxyde metallique et au moins une hydroxyalkyluree**

(30) Priorité: 20.12.2004 FR 0453078
(71) Demandeur: L'ORÉAL, 75008 Paris (FR)
(72) Inventeur: Candau, Didier, 91570 Bievres (FR)
(74) Mandataire: Miszputen, Laurent

(57) **Abrégé**

L'invention concerne une composition comprenant dans un support cosmétiquement acceptable un système filtrant les radiations UV, caractérisée par le fait qu'elle contient :
(a) au moins des pigments minéraux à base d'oxydes métalliques et
(b) au moins une hydroxyalkylurée de formule (I) suivante. 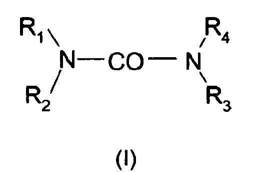
dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que l'un de ses sels, solvats ou isomères; ladite composition ne contenant pas de dipalmitate kojique.

L'invention concerne également l'utilisation d'une hydroxyalkylurée de formule (I) dans une composition comprenant dans un support cosmétiquement acceptable au moins des pigments minéraux à base d'oxydes métalliques, pour réduire voire supprimer l'effet de blanchissement sur la matière kératinique après application.

## Description

L'invention concerne une composition comprenant dans un support cosmétiquement acceptable au moins un système filtrant les radiations UV, caractérisée par le fait qu'elle contient :
(a) au moins des pigments minéraux d'oxyde métallique et
(b) au moins une hydroxyalkylurée particulière.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Les rayons UVA et UVB doivent donc être filtrés et il existe actuellement des compositions cosmétiques protectrices de l'épiderme humain renfermant des filtres UVA et UVB.

Les filtres UV les plus couramment utilisés sont organiques et solubles dans les huiles ou dans les milieux aqueux; ils possèdent généralement dans leur structure un groupe chromophore relié à un groupe solubilisant qui est généralement une chaîne grasse dans le cas des filtres UV liposolubles ou bien un groupe acide carboxylique ou sulfonique dans le cas des filtres UV hydrosolubles.

De nombreuses compositions cosmétiques destinées à la photoprotection de la peau ont été proposées à ce jour et l'utilisation de pigments d'oxydes métalliques tels que les pigments de TiO₂ dans les produits solaires est de plus en plus fréquente car ils permettent d'obtenir des indices de protection très élevés en association avec les filtres UV classiques.

Les compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques lipophiles et/ou des filtres organiques classiques hydrophiles capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Dans de telles émulsions, les filtres hydrophiles sont présents dans la phase aqueuse et les filtres lipophiles sont présents dans la phase grasse.

L'un des inconvénients majeurs des compositions antisolaires connues à ce jour contenant des pigments minéraux d'oxyde métallique en particulier de titane, est que, une fois appliquées sur la peau sous la forme d'un film, elles engendrent sur cette dernière un effet de blanchissement qui est cosmétiquement indésirable et généralement peu apprécié des utilisateurs. Cet effet est d'autant plus marqué que la concentration en pigments dans la composition est élevée. Pour éviter ce problème, il serait bien entendu possible de mettre en oeuvre des quantités réduites de pigments, mails les émulsions résultantes, qui conduiraient certes à une transparence acceptables sur la peau, n'offriraient alors plus de protection convenable dans le domaine des UV, ce qui limite fortement l'intérêt d'une telle opération.

Une autre difficulté réside dans le fait que les émulsions classiques antisolaires à base de pigments protecteurs conduisent, après application à une distribution irrégulière, non homogène voire grossière des pigments sur cette peau, ce qui peut nuire à la qualité de l'effet de photoprotection globale. Cette mauvaise répartition des pigments que l'on constate à la surface de la peau est souvent liée au fait qu'il existe, au niveau de la composition initiale même (avant application), un manque substantiel d'homogénéité (mauvaise dispersion du pigment dans son support).

On a déjà proposé d'inclure des pigments d'oxyde de titane (Tioveil AQ) dans une lotion dépigmentante contenant du dipalmitate kojique. Cette formulation ne permet pas de remédier aux problèmes techniques évoqués ci-dessus.

A la suite d'importantes recherches menées sur la question, il a maintenant été trouvé par la Demanderesse, et ceci de façon tout à fait inattendue et surprenante, qu'il est possible de remédier aux différents inconvénients énumérés ci-dessus, en associant à des pigments minéraux d'oxyde métallique une hydroxyalkylurée de formule (I) que l'on détaillera ci-après. Les compositions antisolaires contenant une telle association présentent en outre une bonne efficacité antisolaire, une bonne rémanence à l'eau, à la transpiration et aux lavages ainsi qu'une bonne persistance dans le temps.

Cette découverte est à la base de la présente invention.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

Ainsi, conformément à l'un des objets de la présente invention, il est maintenant proposé une composition comprenant dans un support cosmétiquement acceptable au moins un système filtrant les radiations UV, caractérisée par le fait qu'elle contient :
(a) au moins des pigments minéraux d'oxyde métallique et
(b) au moins une hydroxyalkylurée de formule (I) que l'on détaillera ci-après ; ladite composition ne contenant pas de dipalmitate kojique.

L'invention concerne également l'utilisation d'une hydroxyalkylurée de formule (I) que l'on détaillera ci-après dans une composition comprenant dans un support cosmétiquement acceptable au moins des pigments minéraux à base d'oxydes métalliques, pour réduire voire supprimer l'effet de blanchissement sur la matière kératinique (telle que la peau, des cils, des sourcils, des ongles ou des muqueuses) après application.

D'autres caractéristiques, aspects et avantages de l'invention apparaîtront à la lecture de la description détaillée qui va suivre.

Les pigments conformes à l'invention ont de préférence une taille moyenne de particule élémentaire supérieure à 5 nm et inférieure à 100 nm. Selon un mode particulièrement préféré de réalisation de l'invention, cette taille varie de préférence de 10 nm à 50 nm.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux des lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme de "silicones" englobe également les silanes nécessaires à leur préparation, en particulier, les alkyl silanes.

Les silicones utilisées pour l'enrobage des nanopigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice tels que le produit "SUNVEIL" de la société IKEDA,
- de silice et d'oxyde de fer tels que le produit "SUNVEIL F" de la société IKEDA,
- de silice et d'alumine tels que les produits "MICROTITANIUM DIOXIDE MT 500 SA" et"MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 T, MT 100 TX, MT 100 Z, MT-01 de la société TAYCA, les produits "Solaveil CT-10 W" et "Solaveil CT 100" de la société UNIQEMA et le produit " Eusolex T-AVO" de la société MERCK,
- de silice, d'alumine et d'acide alginique tel que le produit " MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA,
- d'alumine et traités par une silicone tels que les produits "TIPAQUE TTO-55 (S)" de la société ISHIHARA, ou "UV TITAN M 262" de la société KEMIRA,
   de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres pigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le Ti02 traité par un polydiméthylsiloxane et dont la taille moyenne des particules élémentaires est de 21 nm tel que celui vendu sous la dénomination commerciale "70250 CARDRE UF TiO2Sl3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple
- ceux commercialisés sous la dénomination "Z-COTE" par la société SUNSMART ;
- ceux commercialisés sous la dénomination "NANOX" par la société ELEMENTIS ;
- ceux commercialisés sous la dénomination "NANOGARD WCD 2025" par la société NANOPHASE TECHNOLOGIES ;

Les pigments d'oxyde de zinc enrobés sont par exemple
- ceux commercialisés sous la dénomination "OXIDE ZINC CS-5" par la société Toshibi (ZnO enrobé par polymethylhydrogenesiloxane) ;
- ceux commercialisés sous la dénomination "NANOGARD ZINC OXIDE FN" par la société NANOPHASE TECHNOLOGIES (en dispersion à 40% dans le Finsolv TN, benzoate d'alcools en C₁₂-C₁₅);
- ceux commercialisés sous la dénomination "DAITOPERSION ZN-30" et "DAITOPERSION ZN-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD ULTRAFINE ZNO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "ESCALOL Z100" par la société ISP (ZnO traité alumine et dispersé dans le mélange methoxycinnamate d'ethylhexyle / copolymère PVP-hexadecene / methicone) ;
- ceux commercialisés sous la dénomination "FUJI ZNO-SMS-10" par la société Fuji Pigment (ZnO enrobé silice et polymethylsilsesquioxane) ;
- ceux commercialisés sous la dénomination "NANOX GEL TN" par la société Elementis (ZnO dispersé à 55% dans du benzoate d'alcools en C₁₂-Cₗ₅ avec polycondensat d'acide hydroxystéarique).

Les pigments d'oxyde de cérium non enrobé est vendu sous la dénomination "COLLOIDAL CERIUM OXIDE" par la société RHONE POULENC.
Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Selon l'invention, les pigments d'oxyde de titane, enrobés ou non enrobés, sont particulièrement préférés.

L'oxyde de titane peut se présenter sous forme rutile, anatase ou amorphe, mais de préférence sous forme rutile et/ou anatase et/ou sous une forme amorphe ou substantiellement amorphe.

Les pigments conformes à l'invention représente en général de 0,5 à 40 %, de préférence de 1 à 30 %, du poids total de l'émulsion.

Les hydroxyalkyl urées conforment à l'invention sont choisies par celles répondant à la formule générale (I) : 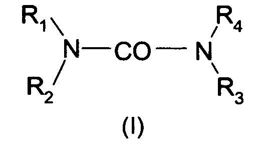 dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.

Dans la formule (I), parmi les groupes alkyle, on peut notamment citer les groupes méthyle, éthyle, n-propyle, isopropyle, n-butyle, iso-butyle et tert-butyle.

Les composés de formule (I) préférés sont ceux ne renfermant qu'un seul groupe hydroxyalkyle, c'est-à-dire ceux pour lesquels R₁ est un groupe hydroxyalkyle et R2, R3 et R4 représentent, indépendamment les uns des autres, un atome d'hydrogène ou un groupe alkyle en C₁-C₄. On préfère plus particulièrement les composés de formule (I) pour lesquels R₁ est un groupe hydroxyalkyle et R₂, R₃ et R₄ représentent chacun un atome d'hydrogène.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle. Le groupe hydroxyéthyle est préféré.
Comme composés de formule (I) préférés, on peut citer la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)- urée; la N-méthyl-N-(1,3,4,5,6-pentahydroxy-2-hexyl)- urée ; la N-méthyl-N'-(1-hydroxy-2-méthyl-2-propyl)- urée ; la N-(1-hydroxy-2-méthyl-2-propyl)- urée ; la N-(1,3-dihydroxy-2-propyl)- urée; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)- urée ; la N,N-Bis-(2-hydroxyéthyl)-N'-propyl- urée; la N,N-Bis-(2-hydroxypropyl)-N'-(2-hydroxyéthyl)- urée ; la N-tert.Butyl-N'-(2-(hydroxyéthyl)-N'-(2-(hydroxypropyl)- urée ; la N-(1,3-dihydroxy-2-propyl)-N'-(2-hydroxyéthyl)- urée; la N,N-Bis-(2-hydroxyéthyl)-N',N'-dimethyl- urée ; la N,N,N',N'-tetrakis-(2-hydroxyéthyl)- urée ; et la N',N'-Bis-(2-hydroxyéthyl)-N',N'-bis-(2-hydroxypropyl)- urée.

Un composé particulièrement préféré pour une utilisation dans la présente invention est la N-(2-hydroxyéthyl)-urée, ci-après désignée par "hydroxyéthyl urée".
Les hydroxyalkyl urées de formule (I) peuvent être préparées comme décrit dans la demande DE-27 03 185. Parmi celles-ci, l'hydroxyéthyl urée est en outre disponible dans le commerce, sous forme de mélange à 50% en poids dans l'eau, auprès de la société NATIONAL STARCH sous la dénomination commerciale Hydrovance®.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Les hydroxyalkylurée conformes à l'invention sont de préférence présentes dans les compositions conformes à l'invention à des teneurs de 0,01 à 50% en poids et plus préférentiellement de 0,1 à 20 % et encore plus préférentiellement de 0,1 à 10% en poids par rapport au poids total de la composition.

Les compositions conformes à l'invention comporteront de préférence d'autres agents photoprotecteurs organiques complémentaires actifs dans l'UVA et/ou l'UVB hydrophiles ou lipophiles ou bien insolubles dans les solvants cosmétiques couramment utilisés.

Les agents photoprotecteurs organiques hydrophiles ou lipophiles complémentaires sont notamment choisis parmi les anthranilates ; les dérivés de dibenzoylméthane ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples d'agents photoprotecteurs organiques complémentaires, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP, Glyceryl PABA,
PEG-25 PABA vendu sous le nom « UVINUL P25 » par BASF,

### Dérivés du dibenzoylméthane :

Butyl Methoxydibenzoylmethane vendu notamment sous le nom commercial « PARSOL 1789 » par HOFFMANN LAROCHE,
Isopropyl Dibenzoylmethane,

### Dérivés salicyligues :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

Dérivés de β,β-diphénylacrylate :
Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

### Dérivés du benzvlidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés du phenyl benzotriazole :

Drometrizole Trisiloxane vendu sous le nom « Silatrizole » par RHODIA CHIMIE ,
Méthylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés de triazine :

Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
2,4,6-tris(4'-amino benzalmalonate de dinéopentyle)-s-triazine
la 2,4,6-tris-(4'-amino benzalmalonate de düsobutyle)-s- triazine.

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Di-néopentyl 4'-méthoxybenzalmalonate
Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

-1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Les agents photoprotecteurs organiques complémentaires préférentiels sont choisis parmi
Ethylhexyl Methoxycinnamate
Homosalate
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s- triazine.
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

Parmi les filtres UV organiques insolubles, on peut citer ceux décrits dans les demandes US 5,237,071, US 5,166,355, GB2303549, DE 197 26 184 et EP893119 et en particulier les dérivés de méthylène bis-(hydroxyphényl benzotriazole) comme le Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent se présenter sous toutes les formes appropriées pour une application topique, notamment sous forme de gels aqueux, sous forme d'émulsions obtenues par dispersion d'une phase grasse (appelée aussi phase huileuse) dans une phase aqueuse (H/E) ou inversement (E/H) ou d'émulsions multiples (par exemple E/H/E ou H/E/H ou H/H/E). Elles peuvent être plus ou moins fluides et avoir l'aspect d'une crème blanche ou colorée, d'une pommade, d'un lait, d'une lotion, d'un sérum, d'une pâte, de poudre, de bâtonnet solide et éventuellement être conditionnée en aérosol et se présenter sous forme de mousse ou de spray. Ces compositions sont préparées selon les méthodes usuelles.

Selon un mode particulier de réalisation de l'invention, la composition selon l'invention se présente sous forme d'une émulsion et comporte alors au moins une phase huileuse. La proportion de la phase huileuse de l'émulsion peut aller de 1 à 80 % en poids, de préférence de 2 à 50 % en poids et mieux de 2 à 40 % en poids par rapport au poids total de la composition. Les corps gras de la phase huileuse, notamment les huiles, et les émulsionnants et co-émulsionnants éventuellement présents, utilisés dans la composition sous forme d'émulsion sont choisis parmi ceux classiquement utilisés dans le domaine cosmétique ou dermatologique. L'émulsionnant et le co-émulsionnant ,quand ils sont présents, le sont généralement, en une proportion allant de 0,1 à 30 % en poids, de préférence de 0,3 à 20 % en poids et mieux de 0,5 à 15 % en poids par rapport au poids total de la composition. L'émulsion peut en outre, contenir des vésicules lipidiques en plus ou à la place des émulsionnants et/ou co-émulsionnants.

Selon leur caractère lipophile, ou au contraire hydrophile, plus ou moins prononcé, les nanopigments pourront être présents soit dans la phase grasse de l'émulsion, soit dans la phase aqueuse, ou bien même encore dans les deux phases à la fois.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; les esters d'acides gras et de sorbitan oxyalkylénés comprenant par exemple de 20 à 100 OE, et par exemple ceux commercialisés sous les dénominations commerciales Tween 20 ou Tween 60 par la société Uniqema ; les éthers d'alcools gras oxyalkylénés (oxyéthylénés et/ou oxypropylénés) ; les esters de sucres, alcoxylés ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100 (nom CTFA : Glyceryl Stéarate / PEG-100 Stéarate) commercialisé sous la dénomination Arlacel 165 par la société Uniqema et sous la dénomination SIMULSOL 165 par la société SEPPIC ; ou le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition, en utilisant des composés appropriés, pour stabiliser lesdites émulsions par exemple des polymères amphiphiles, des charges, des épaississants ou gélifiants.

Quand la composition de l'invention est sous forme d'émulsion, elle comporte au moins une phase huileuse qui contient au moins une huile, notamment une huile cosmétique. On entend par "huile" un corps gras liquide à la température ambiante (25°C).

Comme huiles utilisables dans la composition de l'invention, on peut utiliser par exemple les huiles hydrocarbonées d'origine animale, telles que le perhydrosqualène (ou squalane) ; les huiles hydrocarbonées d'origine végétale, telles que les triglycérides des acides caprylique/caprique comme ceux vendus par la société Stearineries Dubois ou ceux vendus sous les dénominations Miglyol 810, 812 et 818 par la société Dynamit Nobel ou encore les huiles d'origine végétale, par exemple les huiles de tournesol, de maïs, de soja, de courge, de pépins de raisin, de sésame, de noisette, d'abricot, de macadamia, d'arara, de coriandre, de ricin, d'avocat, l'huile de jojoba, l'huile de beurre de karité ; les huiles de synthèse ; les huiles de silicone comme les polyméthylsiloxanes (PDMS) volatiles ou non à chaîne siliconée linéaire ou cyclique, liquides ou pâteux à température ambiante ; les huiles fluorées telles que celles partiellement hydrocarbonées et/ou siliconées comme celles décrites dans le document JP-A-2-295912 ; les éthers tels que l'éther dicaprylique (nom CTFA : Dicaprylyl ether) ; et les benzoates d'alcools gras en C₁₂-C₁₅ (Finsolv TN de FINETEX) ; les dérivés arylalkyl benzoates comme le 2-phenylethyl benzoate (X-Tend 226 de ISP) ; les huiles amidées comme le N-lauroylsarcosinate d'isopropyle (ELDEW SL-205 de Ajimoto) et leurs mélanges.

La phase huileuse peut aussi comporter un ou plusieurs corps gras choisi par exemple parmi les alcools gras (alcool cétylique, l'alcool stéarylique, alcool cétéarylique), les acides gras (acide stéarique) ou les cires (paraffine, cires de polyéthylène, carnauba, cire d'abeilles).

La composition de l'invention peut également contenir un ou plusieurs solvants organiques qui peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des alcools monohydriques linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer les dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Les compositions conformes à la présente invention peuvent également comprendre des adjuvants cosmétiques classiques choisis parmi les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, les charges, les polymères, les propulseurs, les agents alcalinisants ou acidifiants ou tout autre ingrédient habituellement utilisé dans le domaine cosmétique et/ou dermatologique.

Comme épaississants hydrophiles, on peut citer les polymères carboxyvinyliques tels que les carbopols (carbomers) et les Pemulen (Copolymère acrylate/C10-C30-alkylacrylate) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Comme conservateurs, on peut citer les esters de l'acide parahydroxybenzoïque encore appelés Parabens® (en particulier le méthyl parabène, l'éthyl parabène, le propyl parabène), le phénoxyéthanol, les libérateurs de formol comme par exemple l'imidazolidinyl urée ou la diazolidinyl urée, le digluconate de chlorhexidine, le benzoate de sodium, le caprylyl glycol, l'iodo propynyl butyl carbamate, le pentylène glycol, le bromure d'alkyl triméthylammonium tel que le bromure de myristyl-triméthylammonium (nom CTFA : bromure de Myrtrimonium), le bromure de dodécyl-triméthylammonium, le bromure d'hexadécyl-triméthylammonium, et leurs mélanges tel que le mélange vendu sous la dénomination Cetrimide® par la société FEF CHEMICALS.Le conservateur peut être présent dans la composition selon l'invention en une teneur allant de 0,001 à 10 % en poids, par rapport au poids total de la composition, notamment allant de 0,1 à 5 % en poids, et en particulier allant de 0,2 à 3 % en poids.

Comme charges qui peuvent être utilisées dans la composition de l'invention, on peut citer par exemple, les pigments ; la poudre de silice ; le talc ; les particules de polyamide et notamment celles vendues sous la dénomination ORGASOL par la société Atochem ; les poudres de polyéthylène ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres de polymethylmethacrylate telles que celles coomercialisées sous la dénomination MICROPEARL M 100 par la société Matsumoto ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les poudres de polyuréthanne telles que la poudre de copolymère hexaméthylène düsocyanate/trimethylol hexyllactone commercialisée sous la dénomination Plastic Powder D-400 par la société Toshiba Pigment (Nom CTFA : HDI / Trimethylol Hexyllactone Crosspolymer) ; et leurs mélanges. Quand elles sont présentes, ces charges peuvent être en des quantités allant de 0,001 à 20 % en poids, de préférence de 0,1 à 10 % en poids et mieux de 1 à 5 % en poids par rapport au poids total de la composition.

Bien entendu, l'homme de l'art veillera à choisir le ou les éventuels composés complémentaires cités ci-dessus et/ou leurs quantités de manière telle que les propriétés avantageuses attachées intrinsèquement à l'association conforme à l'invention ne soient pas, ou substantiellement pas, altérées par la ou les adjonctions envisagées.

La composition selon l'invention est généralement adaptée à une application topique sur la peau et comprend donc généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.
La composition selon l'invention peut constituer un produit pour le soin de la peau, notamment pour le visage, le cou, le contour de l'oeil, le corps ; ou bien encore un produit de maquillage de la peau tel qu'un produit du teint (notamment fond de teint), un fard à paupières, un fard à joue, un eye-liner, un produit anticernes, un produit de maquillage du corps, un produit de protection solaire ou bien un produit de nettoyage de la peau. De manière préférentielle La composition selon l'invention sera un produit de protection solaire.

La composition est généralement non rincée, mais elle peut être rincée si elle constitue un produit de nettoyage notamment moussant.

L'invention a aussi pour objet un procédé de traitement cosmétique d'une matière kératinique telle que la peau, des cils, des sourcils, des ongles ou des muqueuses, caractérisé en ce qu'on applique sur la matière kératinique, une composition telle que définie ci-dessus.

Selon un autre aspect, l'invention concerne également un ensemble cosmétique comprenant :
i) un récipient délimitant au moins un compartiment, ledit récipient étant fermé par un élément de fermeture ; et
ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Les compositions selon l'invention peuvent se présenter sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

L'invention va maintenant être décrite en référence aux exemples suivants donnés à titre illustratif et non limitatif. Dans ces exemples, sauf indication contraire, les quantités sont exprimées en pourcentages pondéraux. On a réalisé les formulations solaires suivantes ; les quantités sont indiquées en pourcentages en poids :

| **Compositions** | **Ex 1** | **Ex 2** | **Ex 3** | **Ex 4** | **Ex 5** |
|---|---|---|---|---|---|
| **PHASE A** | | | | | |
| Polydiméthylsiloxane | 0,5 | 0,5 | 0,5 | 0,5 | 0,5 |
| Conservateurs | 1,0 | 1,0 | 1,0 | 1,0 | 1,0 |
| Acide stéarique | 1,5 | 1,5 | 1,5 | 1,5 | 1,5 |
| Mélange monostéarate de glycéryle/stéarate-PEG (100 OE) | 1,0 | 1,0 | 1,0 | 1,0 | 1,0 |
| Mélange cetylstéarylglucoside/alcool cetylstéarylique | 2,0 | 2,0 | 2,0 | 2,0 | 2,0 |
| Benzoate d'alcools en C₁₂/C₁₅ | 5,0 | - | 5,0 | 5 ,0 | - |
| Isohexadécane | 1,0 | 1,0 | 1,0 | 1,0 | 1,0 |
| Alcool cétylique | 0,5 | 0,5 | 0,5 | 0,5 | 0,5 |
| Butyl Methoxydibenzoylmethane | 2,0 | 2,5 | 2,0 | 2,0 | 2,0 |
| Octocrylene | 9,0 | 10,0 | 9,0 | 10,0 | 10,0 |
| Drometrizole Trisiloxane | 1,0 | - | 4,0 | - | - |
| Bis-Ethylhexyloxyphenol Methoxyphenyl Triazine | 1,0 | 3,0 | 1,0 | 3,0 | 3,0 |
| Ethylhexyl Methoxycinnamate | - | 3,0 | - | 5,0 | 5,0 |
| TiO₂ | 3,0 | 5,0 | 3,0 | 5,0 | 5,0 |

| **PHASE B** | | | | | |
|---|---|---|---|---|---|
| N-(2-hydroxyléthyl)urée | 5,0 | 10,0 | 5,0 | 5,0 | 10,0 |
| Eau désionisée | qsp 100 | qsp 100 | qsp 100 | qsp 100 | qsp 100 |
| Sequestrant | 0,1 | 0,1 | 0,1 | 0,1 | 0,1 |
| Glycérine | 5,0 | 5,0 | 5,0 | 5,0 | 5,0 |
| Gomme de Xanthane | 0,2 | 0,2 | 0,2 | 0,2 | 0,2 |
| Phosphate de monocétyle | 1,0 | 1,0 | 1,0 | 1,0 | 1,0 |

| **PHASE C** | | | | | |
|---|---|---|---|---|---|
| Copolymère acide acrylique/méthacrylate de stéaryle | 0,2 | 0,2 | 0,2 | 0,2 | 0,2 |
| Triéthanolamine | qs | qs | qs | qs | qs |

On chauffe la phase aqueuse (Phase B) contenant l'ensemble de ses ingrédients à 80°C au bain marie. On chauffe la phase grasse (Phase A) contenant l'ensemble de ses ingrédients à 80°C au bain marie. On émulsionne A dans B sous agitation de type rotorstator (appareil de la société Moritz). On incorpore la phase C et on laisse revenir à température ambiante sous agitation modérée. On introduit la triéthanolamine de façon à ajuster le pH à la valeur désirée en fin de fabrication.

## Revendications

1. Composition comprenant dans un support cosmétiquement acceptable au moins un système filtrant les radiations UV, **caractérisée par le fait qu'**elle contient :
(a) au moins des pigments minéraux d'oxyde métallique
(b) au moins une hydroxyalkylurée de formule (I) 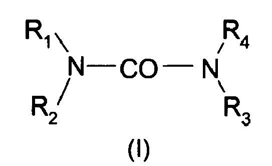 dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que l'un de ses sels, solvats ou isomères ; ladite composition ne contenant pas de dipalmitate kojique.

2. Composition selon la revendication 1, **caractérisée en ce que** R₁ est un groupe hydroxyalkyle et R₂, R₃ et R₄ représentent, indépendamment les uns des autres, un atome d'hydrogène ou un groupe alkyle en C₁-C₄

3. Composition selon la revendication 2, **caractérisée en ce que** R₁ est un groupe hydroxyalkyle et R₂, R₃ et R₄ représentent chacun un atome d'hydrogène.

4. Composition selon l'une quelconque des revendications 1 à 3, **caractérisée en ce que** le composé de formule (I) est choisi parmi la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)- urée ; la N-méthyl-N-(1,3,4,5,6-pentahydroxy-2-hexyl)-urée ; la N-méthyl-N'-(1-hydroxy-2-méthyl-2-propyl)- urée ; la N-(1-hydroxy-2-méthyl-2-propyl)- urée; la N-(1,3-dihydroxy-2-propyl)- urée ; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)- urée ; la N,N-Bis-(2-hydroxyéthyl)-N'-propyl- urée ; la N,N-Bis-(2-hydroxypropyl)-N'-(2-hydroxyéthyl)- urée ; la N-tert.Butyl-N'-(2-(hydroxyéthyl)-N'-(2-(hydroxypropyl)- urée ; la N-(1,3-dihydroxy-2-propyl)-N'-(2-hydroxyéthyl)- urée ; la N,N-Bis-(2-hydroxyéthyl)-N',N'-dimethyl- urée ; la N,N,N',N'-tetrakis-(2-hydroxyéthyl)- urée ; la N',N'-Bis-(2-hydroxyéthyl)-N',N'-bis-(2-hydroxypropyl)- urée.

5. Composition selon l'une quelconque des revendications 1 à 4, **caractérisée en ce que** l'hydroxyalkyl urée est la N-(2-hydroxyéthyl)-urée.

6. Composition selon l'une quelconque des revendications 1 à 5, **caractérisée en** le ou les hydroxyalkylurées sont présentes à des teneurs de 0,01 à 50% en poids et plus préférentiellement de 0,1 à 20 % et encore plus préférentiellement de 0,1 à 10% en poids par rapport au poids total de la composition.

7. Composition selon l'une quelconque des revendications 1 à 6, **caractérisées en ce que** lesdits pigments sont choisis parmi les oxydes de titane, de zinc, de fer, de cérium et leurs mélanges, enrobés ou non.

8. Composition selon la revendication 7, **caractérisée en ce que** les nanopigments sont à base d'oxyde de titane, enrobé ou non enrobé.

9. Composition selon la revendication 8, **caractérisée en ce que** l'oxyde de titane est sous forme rutile, anatase ou amorphe.

10. Composition selon l'une quelconque des revendications 1 à 9, **caractérisée en ce que** pigments ont une taille moyenne de particule élémentaire supérieure à 5 nm et inférieure à 100 nm.

11. Composition selon la revendication 10, **caractérisée en ce que** la taille moyenne de particule élémentaire varie de 10 nm à 50 nm.

12. Composition selon l'une quelconque des revendications 1 à 11, **caractérisée en ce que** la teneur en poids des pigments représente de 0,5 à 40 %, de préférence de 1 à 30 %, du poids total de la composition.

13. Composition selon l'une quelconque des revendications 1 à 12, **caractérisée en ce qu'**elle constitue un produit pour le soin de la peau, un produit de maquillage de la peau, un produit de protection solaire, un produit de nettoyage de la peau.

14. Composition selon la revendication 13, **caractérisée en ce qu'**elle constitue un produit de protection solaire.

15. Procédé de traitement cosmétique d'une matière kératinique, **caractérisé par le fait qu'**on applique sur la matière kératinique, une composition cosmétique selon l'une quelconque des revendications 1 à 14.

16. Ensemble cosmétique comprenant
(i) un récipient délimitant au moins un compartiment, ledit récipient étant fermé par un élément de fermeture ; et
(ii) une composition selon l'une quelconque des revendications 1 à 14 et disposée à l'intérieur dudit compartiment.

17. Utilisation d'une hydroxyalkylurée de formule (I) selon l'une quelconque des revendications précédentes dans une composition comprenant dans un support cosmétiquement acceptable au moins des pigments minéraux à base d'oxydes métalliques, pour réduire voire supprimer l'effet de blanchissement sur la matière kératinique après application.
